# EUROPÄISCHE PATENTSCHRIFT

(11) **EP 3 909 563 B1**
(45) Veröffentlichungstag und Bekanntmachung des Hinweises auf die Patenterteilung: **12.06.2024**
(21) Anmeldenummer: 21172434.9
(22) Anmeldetag: 06.05.2021
(51) Int. Cl.: A61K 8/44, A61K 8/60, A61K 8/67, A61K 8/92, A61Q 19/00

(54) **KOSMETISCHE CREME**
COSMETIC CREAM
CRÈME COSMÉTIQUE

(30) Priorität: 15.05.2020 DE 202020102789 U
(43) Veröffentlichungstag der Anmeldung: 17.11.2021
(73) Patentinhaber: Karpfl, Vera, 49824 Laar (DE); Karpfl, Peter, 49824 Laar (DE)
(72) Erfinder: Karpfl, Frau Dr. Vera, 49824 Laar (DE)
(74) Vertreter: Duda, Rafael Thomas

(56) Entgegenhaltungen:
- EP-B1- 1 595 936
- US-A1- 2017 042 784
- DATABASE GNPD [Online] MINTEL; 24. März 2020 (2020-03-24), anonymous: "Cream", XP055842454, Database accession no. 7460057
- DATABASE GNPD [Online] MINTEL; 23. Januar 2019 (2019-01-23), anonymous: "Eye Cream", XP055813859, Database accession no. 6282809

## Beschreibung

Die vorliegende Erfindung betrifft eine kosmetische Creme zur Aufbringung auf die Haut, enthaltend:
--30 Gew.-% bis 50 Gew.-% Avocadoöl,
--30 Gew.-% bis 55 Gew.-% Wasser,
--5 Gew.-% bis 25 Gew.-% eines oder mehrerer Emulgatoren,
--0,1 Gew.-% bis 1,5 Gew.-% Tocopherol,
--0,1 Gew.% bis 2,0 Gew.-% wenigstens eines Konservierungsmittels,
--0,5 Gew.-% bis 1,2 Gew.-% Folsäure (Vitamain B₉),
--0,5 Gew.-% bis 1,2 Gew.-% Carnitin.

Aus der DE 10 2014 119 283 A1 ist eine kosmetische Wirkstoffkombination zur Behandlung der Haut bekannt, welche das Feuchthaltevermögen der Haut verbessert sowie die Elastizität und Geschmeidigkeit der Haut fördert und einer frühzeitigen Alterung der Haut und Faltenbildung vorbeugen soll. Diese bekannte Wirkstoffkombination enthält als Bestandteile unter anderem Allantoin, Vitamin E und kann Avocadoöl enthalten. Die Wirkstoffkombination kann zur Herstellung von Hautcremes, Hautgels, Hautlotionen oder Hautsalben dienen. Dabei können die Wirkstoffe Allantoin und Vitamin E sowie das Avocadoöl als Feuchtigkeitsspender dienen.

Die DE 10 2006 038 758 A1 beschreibt die kombinierte Anwendung einer Hautcreme und Nahrungsergänzungsmitteln, wobei als Nahrungsergänzungsmittel Vitamin C und Vitamin E genannt werden. Die Inhaltsstoffe dienen der Behandlung von trockener Haut und/oder reifer Haut. Das Nahrungsergänzungsmittel kann weiterhin einen sekundären Pflanzenstoff enthalten und es wird die Verwendung von Vitaminen der B-Reihe erwähnt, nämlich B1, B2, B3, B5 und B6.

Die Verwendung von Kaliumsorbat als Konservierungsmittel in einer kosmetischen Zusammensetzung, die einen feuchtigkeitsspendenden Wirkstoff, Allantoin und Vitamin E enthält und in der Prophylaxe zur Behandlung von zu Akne neigender Haut und zur Behandlung von Hautunreinheiten eingesetzt wird, ist aus der EP 3 217 981 B1 bekannt.

Die Verwendung von Astaxanthin in kosmetischen Zubereitungen wird in dem EP 0 748 625 B1 beschrieben. Aus diesem Dokument ist es bekannt, dass es vorteilhaft sein kann, der Haut, die ultravioletter Strahlung ausgesetzt ist, einer kosmetischen topischen Behandlung zu unterziehen, um sie vor Fotoalterung zu schätzen, wobei man ein dazu fotoumsetzbares Karotinoid verwendet. In dem Dokument wird unter anderem Astaxanthin als Wirkstoff genannt. Die kosmetische Zusammensetzung kann in Form einer Emulsion, eines Gels oder einer Dispersion vorliegen und zur Milderung von Falten und zur Vorbeugung und Bekämpfung von Akne eingesetzt werden.

Die Aufgabe der vorliegenden Erfindung besteht darin, eine kosmetische Creme zur Aufbringung auf die Haut mit den eingangs genannten Merkmalen zur Verfügung zu stellen, welche eine verbesserte Wirksamkeit aufweist und für ein breites Spektrum von Indikationen sowohl im humanen als auch im veterinären Bereich eingesetzt werden kann.

Die Lösung dieser Aufgabe liefert eine kosmetische Creme der eingangs genannten Gattung mit den Merkmalen des Anspruchs 1.

Erfindungsgemäß enthält die kosmetische Creme weiterhin
--einen oder mehrere weitere Wirkstoffe ausgewählt aus der Gruppe umfassend Cyanocobalamin (Vitamin B₁₂), Ubichinon (Coenzym Q₁₀), Allantoin, Astaxanthin, Kurkuma, Nigella sativa (Schwarzkümmel) und Cannabidiol (CBD) in einer Menge von insgesamt 0,5 Gew.-% bis 3 Gew.-%, wobei bei wenigstens zwei aus der genannten Gruppe ausgewählten Wirkstoffen jeder dieser Wirkstoffe in einer Menge von mindestens 0,1 Gew.-% und in einer Menge von höchstens 1,0 Gew.-%, vorzugsweise von höchstens 0,5 Gew.-% enthalten ist.

Cyanocobalamin ist wichtig für die Zellteilung, die Blutbildung und die Funktion des Nervensystems. In jüngerer Zeit wurden Studien mit Vitamin B12-haltigen Salben durchgeführt, die gegen Neurodermitis und Psoriasis wirken sollen, deren Wirksamkeit jedoch umstritten ist. Es wird vermutet, dass die Wirkung auf die Fähigkeit von Cyanocobalamin zurückgeführt wird, Stickstoffmonoxid zu binden, welches bei den symptomatischen Hautveränderungen in erhöhter Konzentration auftreten und zellschädigend wirken soll.

Ubichinon (Coenzym Q₁₀). Ubichinon-10 oder 6-all-trans-Decaprenyl-2,3-dimethoxy-5-methyl-1,4-benzochinon (nach IUPAC) ist ein Elektronen- und Protonen-Überträger in der Atmungskette. Ubichinon wird zum Teil über die Nahrung aufgenommen, aber auch im Körper selbst produziert. Es wird als Nahrungsergänzungsmittel angeboten sowie auch als Bestandteil von kosmetischen Cremes, wobei es den im Alter zunehmenden Mangel an Q-10 ausgleichen und den Abbau von schädlichen Radikalen sicherstellen soll.

Die erfindungsgemäße kosmetische Creme kann wenigstens einen weiteren Wirkstoff ausgewählt aus der Gruppe umfassend Allantoin, Astaxanthin, Kurkuma, Nigella sativa (Schwarzkümmel) und Cannabidiol (CBD) enthalten. Cannabidiol kann als Öl in allen verfügbaren Konzentrationen wie beispielsweise mit einem CBD-Gehalt von 2,5 %, 5 %, 10 %, 15 %, 20 % oder 25 % verwendet werden.

Die Verwendung von Allantoin, mit exaktem Namen N-(2,5-Dioxo-4-imidazolidinyl)-harnstoff, in der Kosmetik, beispielsweise in Hautcremes, Duschgels, Sonnenschutzmitteln, Rasierwässern etc. ist grundsätzlich bekannt. Es wird gegen Hautirritationen eingesetzt, bewirkt die Beschleunigung des Zellaufbaus, die Zellbildung oder Zellregeneration, beruhigt die Haut und unterstützt die Wundheilung.

Bei Astaxanthin oder 3,3'-Dihydroxy-ß,ß-carotin-4,4'-dion handelt es sich um einen natürlichen rötlich-violetten Farbstoff, der zur Klasse der Carotinoide zählt. Es ist grundsätzlich bekannt, dass Astaxanthin eine vitaminartige Wirkung hat und sich positiv auf die Immunabwehr auswirken kann. Aufgrund seiner Absorption und seiner Eigenschaft als Antioxidans kann Astaxanthin die Haut gegen Reizungen schützen, die durch UV-Strahlung ausgelöst werden. Daher wird es auch ergänzend in Sonnenschutzmitteln verwendet.

Kurkuma, auch Gelbwurz genannt, ist eine Pflanzenart aus der Familie der Ingwergewächse, welche neben anderen Stoffen das Curcumin enthält. Es gibt Veröffentlichungen, die Curcumin eine entzündungshemmende Wirkung zuschreiben, wobei es gewisse Enzyme hemmen soll. In der traditionellen Medizin Indonesiens wird Kurkuma ebenfalls in Heilmitteln eingesetzt, wobei es zur Stärkung des Immunsystems beitragen soll. Allerdings ist die Wirkung von Curcumin bislang wissenschaftlich nicht eindeutig belegt und daher in der Fachwelt umstritten. Eine Verwendung von Curcumin in Cremes, die auf die Haut aufgebracht werden, ist bislang nicht üblich.

Nigella Sativa oder Echter Schwarzkümmel ist eine Pflanze aus der Familie der Hahnenfußgewächse. Schwarzkümmel wird seit dem Altertum als Gewürz verwendet sowie auch als Heilmittel gegen diverse Krankheiten. In jüngeren medizinischen Veröffentlichungen werden dem Schwarzkümmelöl antibakterielle Wirkungen zugeschrieben, die auf das Thymochinon zurückgehen sollen. Die Verwendung von reinem Schwarzkümmelöl in der Naturheilkunde bei Allergien, Neurodermitis oder Psoriasis ist grundsätzlich bekannt.

Cannabidiol (CBD) ist ein Wirkstoff aus der Gruppe der Cannabinoide. Es handelt sich um einen natürlichen Inhaltsstoff des Hanfs, welcher aber anders als Tetryhydrocannabinol keine psychoaktive Wirkung hat. Cannabidiol wird in Nahrungsergänzungsmitteln und Kosmetika verwendet und hat unter anderem entzündungshemmende und antioxidative Eigenschaften. Die topische Verabreichung von Cannabidiol ist grundsätzlich bekannt. Cannabidiol-Öl ist in verschiedenen Konzentrationen erhältlich, wobei beispielsweise ein 10 %iger Extrakt einen CBD-Gehalt von 1000 mg aufweist.

L-Carnitin oder (3-Carboxy-2-hydroxypropyl)-N,N,N-trimethylammoniumhydroxid ist eine chemische Verbindung, die eine Rolle im Energiestoffwechsel von Fettsäuren spielt. Die Verwendung von Carnitin als Nahrungsergänzungsmittel ist bekannt und es wird in Shampoos gegen Haarausfall vermarktet. Bei der Verwendung in Cremes soll Carnitin unterstützend auf den Abbau von Cellulitis wirken.

Folsäure, auch bekannt unter der Bezeichnung Vitamin B₉, ist für den menschlichen Körper essentiell und muss mit der Nahrung aufgenommen werden. Folsäure findet sich beispielsweise in Hefen, Kleien und Hülsenfrüchten. Folsäure erfüllt im menschlichen Organismus diverse wichtige Funktionen und ist unter anderem an der Synthese der DNA und RNA beteiligt und reguliert wichtige Stoffwechselwege. In der Kosmetik wird Folsäure verwendet, um den Feuchtigkeitslevel der Haut zu verbessern, die Kollagenbildung zu fördern und den transepidermalen Wasserverlust zu verringern. Daher wird Folsäure eine Anti-Aging-Wirkung für die menschliche Haut zugeschrieben.

Gemäß einer bevorzugten Weiterbildung der vorliegenden Erfindung enthält die erfindungsgemäße Hautcreme vorzugsweise weiterhin Dimethylsulfoxid (DMSO). Dimethylsulfoxid ist als Wirkstoff aus der Gruppe der Antiphlogistika bekannt und wird zur äußerlichen Behandlung von Schmerzen, Entzündungen und Schwellungen eingesetzt. DMSO hat entzündungshemmende, antioxidative, schmerzstillende, gefäßerweiternde, wundheilungsfördernde und antimikrobielle Eigenschaften und erhöht die Aufnahme pharmazeutischer Wirkstoff in die Haut. Die Verwendung von DMSO in Gels, Cremes und Sprays, die auf die Haut aufgebracht werden, ist bekannt.

Die erfindungsgemäße kosmetische Hautcreme enthält Avocadoöl als pflanzliches Öl, welches in einem Anteil von 30 Gew.-% bis 50 Gew.-%, bevorzugt in einem Anteil von insgesamt 35 Gew.-% bis 45 Gew.-% in der kosmetischen Creme enthalten ist.

Avocadoöl ist ein Pflanzenöl, das aus den Früchten des Avocadobaums gewonnen wird. Es handelt sich um ein Triglycerid, das heißt ein Gemisch verschiedener Triester des Glycerins mit wechselnden Anteilen von Fettsäureresten, wobei der Ölsäurerest einen vergleichsweise hohen Anteil von ca. 52 % hat. Bei Ölsäure handelt es sich um die einfach ungesättigte Octadecensäure. Außerdem enthält Avocadoöl auch Phytosterine. Die Verwendung von Avocadoöl in Hautpflegeprodukten ist an sich bekannt. Es hat den Vorteil, dass es von der Haut gut aufgenommen wird, schnell einzieht und aufgrund der enthaltenen Vitamine A und E sowie der ungesättigten Fettsäuren das Hautbild verbessert.

Gemäß einer bevorzugten Weiterbildung der vorliegenden Erfindung enthält die erfindungsgemäße Hautcreme als Konservierungsmittel vorzugsweise Natriumsorbat oder Kaliumsorbat. Die genannten Alkalisalze der Sorbinsäure eignen sich insbesondere aufgrund ihrer Hemmung der Bildung von Hefen und Schimmel sowie auch einiger Bakterien gut als Konservierungsmittel. Ebenfalls vorteilhaft ist die sehr leichte Wasserlöslichkeit dieser Salze der Sorbinsäure, da die Grundlage der erfindungsgemäßen Hautcreme neben den oben genannten pflanzlichen Ölen einen hohen Wasseranteil von 30 Gew.-% bis 55 Gew.-% enthält, d.h. bis zu etwa der Hälfte der Salbengrundlage Wasser ist, welches mit Hilfe wenigstens eines Emulgators mit dem Pflanzenöl emulgiert wird.

Gemäß einer bevorzugten Weiterbildung der vorliegenden Erfindung enthält die erfindungsgemäße kosmetische Creme wenigstens einen nichtionischen Öl-Wasser-Emulgator auf pflanzlicher Basis, insbesondere Methylglucosesesquistearat. Diese Substanz eignet sich gut für die Bildung stabiler Formulierungen von Ölen und Fetten mit diversen Wirkstoffen und Salzen und ist besonders als Emulgator für Cremes gut geeignet. Methylglucosesequistearat ermöglicht die Bildung fein verteilter Mischungen von Öl und Wasser, ist aufgrund der pflanzlichen Herkunft im Hinblick auf die Toxizität unbedenklich und hat zudem eine hautpflegende Wirkung.

Gemäß einer bevorzugten Weiterbildung der vorliegenden Erfindung enthält die erfindungsgemäße kosmetische Creme Propylenglykol, insbesondere in einer Menge von wenigstens 5 Gew.-%. Propylenglykol oder 1,2-Propandiol kann in der Hautcreme als Feuchthaltemittel dienen und es hat außerdem eine antimikrobielle Wirkung, so dass es auch als Konservierungsmittel dient. Darüber hinaus kann durch das Propylenglykol die Resorption der Wirkstoffe verbessert werden und das Propylenglykol fördert die Bildung einer Wasser-in-Öl-Emulsion, das heißt es wirkt auch als ein zusätzlicher Emulgator.

Die erfindungsgemäße Creme ist konzipiert als Hautpflegeprodukt (Kosmetik) und ist besonders gut geeignet für die empfindsame Haut, wobei ihre Wirkung auf den fünf Grundsäulen der Zellerhaltung und -regeneration basiert und sie sich durch Anwendungsvielfalt und vielseitige Verwendungsmöglichkeiten auszeichnet.

Zusätzlich zu ihrer rein natürlich pflegenden Eigenschaft, konnten für die erfindungsgemäße kosmetische Creme folgende Parameter erarbeitet werden.

Sie wirkt schmerzlindernd, wundheilungsfördernd, Juckreiz stillend, entzündungshemmend, blutstillend, heilungsfördernd, fördert die Zellregeneration und wirkt abschwellend.

Anwendungsgebiete für die erfindungsgemäße kosmetische Creme sind insbesondere Hautprobleme und weitere Indikationen der verschiedensten Art.

Die erfindungsgemäße kosmetische Creme ist insbesondere geeignet zur vorbeugenden Behandlung, Behandlung oder Pflege von Hautkrankheiten bei Menschen oder Säugetieren, insbesondere zur Wundheilung, Anwendung bei Allergien, bei Verletzungen, bei Muskelschmerzen oder -verletzungen, bei Schwellungen, bei Verbrennungen, Sonnenbrand, Hautreizungen, Schnittverletzungen, Wundheilungsstörungen, zur Regeneration des Milieus bei Pilzinfektionen, bei der Säuglingspflege (insbesondere Windeldermatitis), bei Akne, bei Hämorrhoiden, zur Narbentherapie nach Operationen, bei Gelenkproblemen, Entzündungen, Zahnfleischerkrankungen, Augenentzündungen (äußerlich angewendet), verdünnt in einem Wasserbad bei Gelenkschmerzen im Fußbereich, bei Hämorrhoiden (inwendig sowie äußerlich), frischen Wunden, Schuppenflechte und Neurodermitis oder zur Pflege bei Ichthyosis.

Die erfindungsgemäße Creme ist ein reines Naturprodukt und kann sogar mit dem Hinweis "vegan" versehen werden.

Durch zwei Studien in Münster an der Universität im Bereich der Dermatologie konnte nachgewiesen werden, dass die erfindungsgemäße Creme eine generelle Verträglichkeit besitzt und im Wesentlichen nebenwirkungsfrei ist. Dies ist in der Rezeptierung eine wichtige Voraussetzung vor allem bei der Anwendung auf bereits gereizter Haut (Anwendungsprobanden waren Neurodermitiker).

Die Haut ist das größte Organ des Menschen und stellt nicht nur äußerlich eine Barriere und ein Empfindungsorgan dar, sondern ist die äußerliche Darstellung aller Lebensprozesse auch im inwendigen unsichtbaren Anteil des Körpers (sowohl bei Menschen als auch bei Tieren). Folgende fünf grundlegende Funktionen der Lebensprozesse werden auf der Haut sichtbar:
- Stoffwechsel,
- Atmung,
- Kommunikation,
- Nervensystem,
- und die Bewegung.

Alle eingangs genannten Inhaltsstoffe der kosmetischen Creme sind bereits zugelassene Wirkstoffe, was für die Vermarktung von Vorteil ist, da keine aufwändigen klinischen Versuchsreihen notwendig sind, wie bei vollkommen neuartigen Wirkstoffen.

Die erfindungsgemäße Creme ist sehr vielseitig und hat einen großen Anwendungsund Wirkmechanismus, bedingt durch die spezifische Kombination der genannten Wirkstoffe. Je nach Indikation kann die oben angeführte beispielhafte Rezeptur mit den dort genannten sechs Wirkstoffen durch Kombination mit einem oder mehreren weiteren Wirkstoffen variiert werden.

Als Wirkstoffe in der Rezeptur der kosmetischen Creme, die in unterschiedlichen Mengenanteilen beigemischt werden können, kommen in Betracht:
CBD (in allen Prozenten 2,5, 5, 10, 15, 20, 25 %ig)
Astaxanthin
DMSO
Kurkuma
Nigella sativa

Die Anwendung der erfindungsgemäßen kosmetischen Creme kann entweder im humanen oder im veterinären Bereich erfolgen. Die jeweiligen Anwendungsmodalitäten sind dabei im Veterinärbereich die gleichen wie im Humanbereich.

Gemäß der vorliegenden Erfindung enthält eine beispielhafte kosmetische Hautcreme die in der nachfolgend genannten Herstellungsanweisung angeführten Inhaltsstoffe:
Es ist noch zu erwähnen, dass gemäß einer optionalen Weiterbildung der Erfindung bei allen Variationen der erfindungsgemäßen Creme die Anreicherung über Ozon erfolgen kann.

Nachfolgend wird die folgende Erfindung anhand eines weiteren konkreten Ausführungsbeispiels näher erläutert.

### HERSTELLUNGSANWEISUNG

für die erfindungsgemäße kosmetische Creme mit der in der nachfolgenden Tabelle angegebenen Zusammensetzung

| Nr | Rohstoff | Prozent | 10.000g | |
|---|---|---|---|---|
| 1 | Avocadoöl | 32.00 | 3.200,0 | |
| 2 | Avocadoöl | 5,90 | 590,0 | |
| 3 | Tegocare PS | 6,00 | 600,0 | |
| 4 | Wasser | 33,30 | 3.300,0 | |
| 5 | Wasser | 8,79 | 879,0 | |
| 6 | Propylenglykol | 10,00 | 1.000,0 | |
| | | | | |
| 7 | Zitronensäure | 0,25 | 25,0 | |
| 8 | Kaliumsorbat | 0,26 | 26,0 | |
| 9 | L-Carnitin | 0,80 | 80,0 | |
| 10 | Cyanocobalamin | 0,10 | 10,0 | |
| | | | | |
| 11 | Folsäure | 0,70 | 70,0 | |
| 12 | Coenzym Q10 | 0,90 | 90,0 | |
| 13 | Vitamin E | 1,00 | 100,0 | |

### Produktionsanleitung

### Phase A:

- Avocadoöl (1) wird in den Schmelzbehälter gegeben
- Tegocare PS (3) (es handelt sich um einen Emulgator, hergestellt von der Evonik Nutrition & Care GmbH, welcher Methylglukosesesquistearat enthält) hinzugeben und bei einer Temperatur von 65-85° schmelzen

### Phase B:

- Wasser (4) und Propylenglykol (6) werden auf 65-85° erwärmt
- In das erwärmte Propylen-Wasser-Gemisch werden Zitronensäure (7), Kaliumsorbat (8), L-Carnitin (9) und Cyanocobalamin (10) hinzugegeben

### Phase C:

- Wasser (5) auf 45-55° erwärmen und Folsäure (11) darin lösen

### Phase D:

- Avocadoöl (2) auf 45-55°C erwärmen und Coenzym Q10 (12) darin lösen
- Vitamin E (13) hinzugeben
   1. Der Doppelmantel der Maschine wird mit heißem Wasser gefüllt (Wasser laufen lassen)
   2. Die Phase A wird in den Kessel gegeben und unter Vakuum (ca 0,8 bar/11,6 psi) 60 Sekunden bei 3.000 rpm homogenisiert
   3. Der Doppelmantel wird nun mit kaltem Wasser gefüllt (Wasser laufen lassen) und unter Vakuum 5 Minuten bei 1.500 rpm gerührt
   4. Hat der Inhalt des Kessels eine Temperatur von ca. 45°C so wird Phase C hinzugegeben und unter Vakuum 60 Sekunden bei 3.000 rpm homogenisiert
   5. Nun gibt man Phase D hinzu und homogenisiert unter Vakuum 60 Sekunden bei 3.000 rpm

## Patentansprüche

1. Kosmetische Creme zur Aufbringung auf die Haut, enthaltend:
-- 30 Gew.-% bis 50 Gew.-% Avocadoöl,
--30 Gew.-% bis 55 Gew.-% Wasser,
--5 Gew.-% bis 25 Gew.-% eines oder mehrerer Emulgatoren,
--0,1 Gew.-% bis 1,5 Gew.-% Tocopherol,
--0,1 Gew.% bis 2,0 Gew.-% wenigstens eines Konservierungsmittels,
--0,5 Gew.-% bis 1,2 Gew.-% Folsäure (Vitamin B₉),
--0,5 Gew.-% bis 1,2 Gew.-% Carnitin,
--einen oder mehrere weitere Wirkstoffe ausgewählt aus der Gruppe umfassend Cyanocobalamin (Vitamin B₁₂), Ubichinon (Coenzym Q₁₀), Allantoin, Astaxanthin, Kurkuma, Nigella sativa (Schwarzkümmel) und Cannabidiol (CBD) in einer Menge von insgesamt 0,5 Gew.-% bis 3 Gew.-%, wobei bei wenigstens zwei aus der genannten Gruppe ausgewählten Wirkstoffen jeder dieser Wirkstoffe in einer Menge von mindestens 0,1 Gew.-% und in einer Menge von höchstens 1,0 Gew.-%, vorzugsweise von höchstens 0,5 Gew.-% enthalten ist.

2. Kosmetische Creme nach Anspruch 1, **dadurch gekennzeichnet**, **dadurch gekennzeichnet, dass** diese Dimethylsulfoxid (DMSO) enthält.

3. Kosmetische Creme nach einem der Ansprüche 1 oder 2, **dadurch gekennzeichnet, dass** diese als Konservierungsmittel Natriumsorbat oder Kaliumsorbat enthält.

4. Kosmetische Creme nach einem der Ansprüche 1 bis 3, **dadurch gekennzeichnet, dass** diese wenigstens einen nichtionischen Öl-Wasser-Emulgator auf pflanzlicher Basis, insbesondere Methylglucosesesquistearat enthält.

5. Kosmetische Creme nach einem der Ansprüche 1 bis 4, **dadurch gekennzeichnet, dass** diese Propylenglykol enthält, insbesondere wenigstens 5 Gew.-% Propylenglykol enthält.

## Claims

1. A cosmetic cream for application to the skin, containing:
- 30 % by weight to 50 % by weight of avocado oil,
- 30 % by weight to 55 % by weight of water,
- 5 % by weight to 25 % by weight of one or more emulsifying agents,
- 0.1 % by weight to 1.5 % by weight of tocopherol,
- 0.1 % by weight to 2.0 % by weight of at least one preservative,
- 0.5 % by weight to 1.2 % by weight of folic acid (vitamin B₉),
- 0.5 % by weight to 1.2 % by weight of carnitine,
- one or more further active ingredients selected from the group comprising cyanocobalamin (vitamin B₁₂), ubiquinone (coenzyme Q₁₀), allantoin, astaxanthin, curcuma, nigella sativa (black cumin) and cannabidiol (CBD) in a total quantity of 0.5 % by weight to 3 % by weight, wherein for at least two active ingredients selected from the cited group, it contains each of these active ingredients in a quantity of at least 0.1 % by weight and in a quantity of at most 1.0 % by weight, preferably at most 0.5 % by weight.

2. The cosmetic cream as claimed in claim 1, **characterized in that** it contains dimethylsulphoxide (DMSO).

3. The cosmetic cream as claimed in one of claims 1 or 2, **characterized in that** it contains sodium sorbate or potassium sorbate as the preservative.

4. The cosmetic cream as claimed in one of claims 1 to 3, **characterized in that** it contains at least one plant-based, non-ionic oil-water emulsifying agent, in particular methyl glucose sesquistearate.

5. The cosmetic cream as claimed in one of claims 1 to 4, **characterized in that** it contains propylene glycol, in particular at least 5 % by weight of propylene glycol.

## Revendications

1. Crème cosmétique, destinée à une application cutanée, contenant :
-- de 30 % en poids à 50 % en poids d'huile d'avocat,
-- de 30 % en poids à 55 % en poids d'eau,
-- de 5 % en poids à 25 % en poids d'un ou de plusieurs agents émulsifiants,
-- de 0,1 % en poids à 1,5 % en poids de tocophérol,
-- de 0,1 % en poids à 2,0 % en poids d'au moins un agent conservateur,
-- de 0,5 % en poids à 1,2 % en poids d'acide folique (vitamine B₉),
-- de 0,5 % en poids à 1,2 % en poids de carnitine,
-- un ou plusieurs principes actifs additionnels, sélectionnés dans le groupe comprenant la cyanocobalamine (vitamine B₁₂), l'ubiquinone (coenzyme Q₁₀), l'allantoïne, l'astaxanthine, le curcuma, la nigella sativa (cumin noir) et le cannabidiol (CBD) dans une quantité totale de 0,5 % en poids à 3 % en poids, pour au moins deux des principes actifs sélectionnés dans le groupe cité, chacun desdits principes actifs étant contenu dans une quantité d'au moins 0,1 % en poids et dans une quantité d'au plus 1,0 % en poids, de préférence d'au plus 0,5 % en poids.

2. Crème cosmétique selon la revendication 1, **caractérisée en ce qu'**elle contient du diméthylsulfoxide (DMSO).

3. Crème cosmétique selon l'une quelconque des revendications 1 ou 2, **caractérisée en ce qu'**elle contient en tant qu'agent conservateur du sorbate de sodium ou du sorbate de potassium.

4. Crème cosmétique selon l'une quelconque des revendications 1 à 3, **caractérisée en ce qu'**elle contient au moins un émulsifiant huile en eau non ionique sur base végétale, notamment le **méthyl glucose** sequistearate.

5. Crème cosmétique selon l'une quelconque des revendications 1 à 4, **caractérisée en ce qu'**elle contient du propylène glycol, notamment au moins 5 % en poids de propylène glycol.
